# EUROPEAN PATENT APPLICATION

(11) **EP 3 459 930 A1**
(43) Date of publication of application: **27.03.2019**
(21) Application number: 17193248.6
(22) Date of filing: 26.09.2017
(51) Int. Cl.: C07C 263/04, C07C 265/14, C07C 269/04, C07C 271/20, C07C 271/52

(54) **MULTISTEP PROCESS FOR THE PREPARATION OF HEXAMETHYLENE DIISOCYANATE, PENTAMETHYLENE DIISOCYANATE OR TOLUENE DIISOCYANATE**

(71) Applicant: Covestro Deutschland AG, 51373 Leverkusen (DE)
(72) Inventor: The designation of the inventor has not yet been filed
(74) Representative: Davepon, Björn

(57) **Abstract**

The present invention relates to a multistep process for the preparation of organic diisocyanates by converting the corresponding diamine precursors, urea and hydroxy compounds into monomeric diurethanes, converting these diurethanes into diurethanes of high boiling hydroxy compounds, and finally cleavage of the latter diurethanes to form the diisocyanates and recover the high boiling hydroxy compounds.

## Description

The present invention relates to a multistep process for the preparation of organic diisocyanates by converting the corresponding diamine precursors, urea and hydroxy compounds into monomeric diurethanes, converting these diurethanes into diurethanes of high boiling hydroxy compounds, and finally cleavage of the latter diurethanes to form the diisocyanates and recover the high boiling hydroxy compounds.

The industrial processes for the preparation of organic diisocyanates, be it aromatic, aliphatic or cycloaliphatic disocyanates, are commonly based on phosgenation of the corresponding diamines. There have been numerous efforts to avoid use of phosgene in the synthesis of the organic diisocyanates not only due to the toxicity of phosgene, but also in order to avoid producing large quantities of hydrogen chloride as a byproduct.

The most common phosgene free route for the production of isocyanates is the thermal cleavage of the corresponding urethanes that yields alcohols and isocyanates. It has been described numerous times. For example EP 1 512 682 A1 describes a multi stage process for the production of cycloaliphatic diisocyanates. In the first stage, a diurethane is formed from the reaction of diamine, carbonic acid derivative, and a hydroxy compound. The hydroxy compound is an alcohol having boiling points below 190 °C at normal pressure and preferably it is 1-butanol. After purification of the obtained diurethane, it is thermally cleaved in a second stage to obtain the cycloaliphatic diisocyanate and a hydroxy compound.

In order to suppress recombination of hydroxy compound and isocyanate, a highly quantitative separation of the thermal cleavage products is desirable. To achieve this, US 5,386,053 describes the use of hydroxy compounds having boiling points that are sufficiently far from the boiling point of the diisocyanate. Thus, preference is given to aliphatic hydroxy compounds, and in particular to n-butanol and/or isobutanol which have boiling points far below the boiling points of industrially relevant diisocyanates. However, when using such low boiling hydroxy compounds, the hydroxy compounds will be obtained as a distillate and the isocyanate will be the bottom product when the crude product is refined in a distillation step. Therefore, it will still contain high boiling impurities, like carbamic acid alkyl esters (urethanes). Furthermore, the use of catalysts for the thermolytic cleavage reaction becomes difficult in this setup because catalyst will be entrained in the isocyanate where it may facilitate reactions of the iscocyanate groups and reduce shelf life of the product. If this bottom product is distilled again in a downstream process step after removal of the hydroxy compound, the isocyanate is in the distillate fraction. Nevertheless it will be difficult to achieve high purity, because the high boiling impurities can cleave and release the low boiling hydroxy compounds which again would be part of the distillate and recombine with the diisocyanate.

Recently, in EP 2 679 575 A1, a process has been described that comprises the step of subjecting an N-substituted carbamic acid ester to a thermal cleavage reaction. It is described that aromatic hydroxy compounds are preferred hydroxy compounds for the formation of the carbamic acid ester which results in the formation of carbamic acid-O-aryl esters (arylurethanes) that undergo thermal cleavage more easily compared to carbamic acid-O-alkyl esters. The aromatic hydroxy compounds mentioned in the document include for example t-octylphenol, 2,4-di-t-amylphenol or p-cumylphenol which have boiling points higher than the boiling points of industrially relevant diisocyanates like hexamethylene diisocyanate, pentamethylene diisocyanate, or isophorone diisocyanate. This combination allows thermal cleavage and subsequent distillation with the isocyanate being the distillate rather than the bottom product so that efficient separation of isocyanate and hydroxy compound is possible.

For the formation of the O-arylurethanes, it is desirable to use high stoichiometric excess of the hydroxy compound based on the amount of amino groups of the organic primary amine used. A preferred range of 2:1 to 50:1 is mentioned. This leads to very high mass flows of the hydroxy compound which is linked to high energy consumptions for conveying, heating, evaporating, condensing and cooling the hydroxy compound in the course of the process. Furthermore, the substituted aromatic hydroxy compounds are of limited stability under the reaction conditions, leading to losses and additional efforts for treating, disposing or recycling the decomposition products.

EP 0 320 235 A2 describes the formation of aliphatic O-arylurethanes that can be used as precursors for isocyanates. Preference is given to mono hydroxy compounds and particularly to phenols having low boiling points as the aromatic hydroxy compounds in order to achieve easy separation. However, this preference for phenol comes along with the above mentioned problems of isocyanate purification and restrictions in the use of catalysts.

EP 2 679 575 A1 and EP2088137 B1 both describe a transesterification step that allows conversion of dialkylurethanes or diaralkylurethanes into diarylurethanes. The main purpose of the transesterification in these documents is the formation of diarylurethanes which allow thermal cleavage reaction to the diisocyanate at milder conditions and with less byproducts than the dialkyl-or diaralkylurethanes. A preference for higher boiling alcohols that allow better purification of the isocyanate is not described.

Therefore, it was an object of the present invention to provide a phosgene free and efficient multistep process for preparing organic diisocyanates which allows fast separation of the isocyanate from the hydroxy compound in the form of a distillate.

This object was solved by a process for preparing hexamethylenediisocyanate, pentamethylenediisocyanate or toluenediisocyanate comprising the following steps:
(I) Reacting a primary diamine of the general formula (1), urea and a hydroxy compound of the general formula (2) with removal of ammonia to a diurethane of the general formula (3),

   H₂N-R-NH₂ (1)

   R'-OH (2)

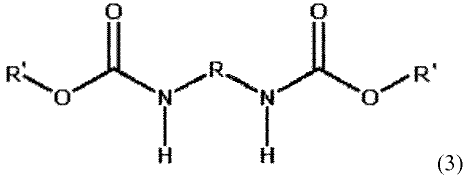

   wherein
   - R: represents a bivalent hydrocarbon radical which can be derived from 2,4-toluene diisocyanate, 2,6-toluene diisocyanate, hexamethylene diisocyanate or pentamethylene diisocyanate by removing the two isocyanate groups,
   - R': represents a hydrocarbon radical which can be derived from an aliphatic or an aromatic hydroxy compound with a standard boiling point ≤ 230 °C by removing the OH group,
(II) Converting the diurethane obtained in step (I) to a diurethane of the general formula (4) by a transesterification reaction with a hydroxy compound of the general formula (5),

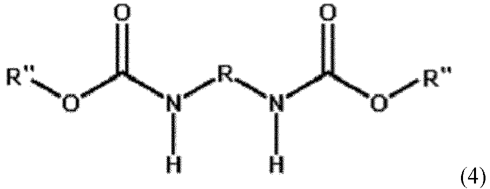

   R"-OH (5)

   wherein
   - R: is the same as above,
   - R": represents a hydrocarbon radical which can be derived from an aliphatic or an aromatic hydroxy compound with a standard boiling point ≥ 280 °C by removing the OH group,
(III) Subjecting the diurethane of the general formula (4) to a thermal cleavage reaction to prepare the diisocyanate of the general formula (6)

   OCN-R-NCO (6)

   wherein
   R is the same as above,
   and the hydroxy compound of the general formula (5)
(IV) Separating the diisocyanate of the general formula (6) from the hydroxy compound of the general formula (5) by distillation.

The process of the present invention allows production hexamethylenediisocyanate, pentamethylenediisocyanate or toluenediisocyanate in high purity. On the one hand, the temperature limitations allow good separation of R'-OH in the transesterification step which allows to push the equilibrium reaction to the side of the diurethane of the general formula (4). On the other hand, the R"-OH can easily be separated as a high boiler from the diisocyanate during or after the cleavage reaction.

In a preferred embodiment of the invention, the molecular mass of R'-OH is at least 40 g/mol lower, preferably at least 100 g/mol lower and most preferably between ≥110 g/mol and < 160 g/mol lower than the molecular mass of R"-OH. This difference in molecular weight is advantageous with regard to the processing cost of the overall process.

The formation of the diurethanes of the general formula (3) from organic diamines, urea and hydroxy compounds has been described in the literature and the procedures can be adapted by the skilled artisan for the formation of the desired diurethane compounds. The formation can proceed either in a one-step process from the three starting materials (see for example EP 2 679 575 A1, example 16 or US5,386,053A, example 1) or in a two-step process with interim formation of the corresponding diurea compound (see for example EP 2 679 575 A1, example 18, steps 18-1 and 18-2).

It is preferred to carry out the reaction using a distillation column. The advantages of such an embodiment over other possible reactor types are a pre-purification of the produced diurethanes and at least a partial removal of low boiling byproducts that can be formed during the reaction and/or excess starting materials. The diurethane can be obtained in good yield at a discharge port being located at the bottom of the column. The distillate is preferably cooled in a way that allows partial condensation of the distillate. The liquids are then recycled to the feed of the column in order to optimize the use of starting materials and reduce material consumption, while a gaseous stream containing low boiling byproducts is unloaded from the process. These byproducts can later on be recovered from the gas stream, for example by condensation or absorption, for further commercial use, incineration or disposal.

If the formation of the diurethanes is done in a two-step process, parts of the starting material are pre-reacted preferably at mild conditions before conversion to the diarylurethanes is performed. During the abovementioned pre-reaction, urea is present and the organic diamine and/or the aromatic hydroxy compound. The pre-reaction preferably takes place at temperatures below 180 °C, more preferably below 150 °C. In another preferred embodiment, released ammonia is removed from the reaction via the gaseous phase, thereby driving reaction to completion. Suitable reactors for the pre-reaction step are for example stirred vessels, but also other reactor types can be utilized.

The products of the pre-reaction stage, or, in case of a one-step synthesis the organic diamine and urea, are then converted with the hydroxy compound R'-OH, preferably in presence of urea, to the diarylurethane of the general formula (3) at a reaction temperature between 180 and 280 °C, preferably between 200 and 260 °C and most preferably between 200 and 240 °C.

The gross reaction scheme for the formation of the diurethanes of the general formula (3) is as follows:

H₂N-R-NH₂ + 2H₂N-CO-NH₂ + 2R'-OH → R'-O-CO-NH-R-NH-CO-O-R' + 4NH₃,

wherein R and R' are as defined above.

Preferred diamines for the formation of the diurethanes of the general formula (3) are 1,5-pentanediamine (PDA) or 1,6-hexanediamine (HDA) which can be used to produce pentamethylene diisocyanate or hexamethylene diisocyanate, respectively, according to the process of the present invention. The most preferred diamine is HDA, because of its high industrial relevance, its high thermal stability and the moderate reactivity of the corresponding diisocyanate hexamethylene diisocyanate (HDI).

The hydroxy compound R'-OH used for the formation of the diurethanes of the general formula (3) is a hydroxy compound that has a standard boiling point ≤ 230 °C. The standard boiling point of a compound is defined according to the IUPAC definition as the temperature at which boiling of the compound occurs under a pressure of 1 bar. In a preferred embodiment of the invention, such hydroxy compounds are used for the formation of the diurethanes of the general formula (3) that have a standard boiling point between ≥ 80 °C and ≤ 210 °C, preferably between ≥ 100 °C and ≤ 190 °C, more preferably between ≥110 °C and ≤160 °C and most preferably between ≥ 115 °C and ≤ 140 °C. Such hydroxy compounds have favorable vapor pressures for the inventive process which allows on the one hand good separation as vapors in the transesterification step and on the other hand easy condensation of the vapors even at vacuum conditions. Particularly suitable hydroxy compounds are n-butanol, 3-methyl-1-butanol or phenol because of their good ability to solubilize urea at higher temperatures. Out of these, 3-methyl-1-butanol and butanol have the advantage of being liquid at ambient temperatures.

The conversion of the diurethane compound of the general formula (3) into the diurethane of the general formula (5) is done in a transesterification reaction, in the following simply named transesterification. This transesterification is a reaction of the diurethane compound of the general formula (3) with a hydroxy compound R"-OH that leads to a replacement of the R'-O groups in the urethane with R"-O groups, resulting in the formation of the diurethane of the general formula (5) and the hydroxy compound R'-OH.

The transesterification can in principle be carried out as described, for example, in [0054]-[0061] of EP 2 088 137 B1 for the transesterification of dialkylurethanes with aromatic hydroxy compounds into diarylurethanes. This process is also applicablable when aliphatic hydroxy compounds having a high standard boiling point are used as hydroxy compounds.

In a preferred embodiment of the invention, the hydroxy compound R"-OH is used in an amount that the number of OH groups is higher than that of the urethane groups in the reaction mixture. In a more preferred embodiment, R"-OH is used in an amount that the number of OH groups is between 2 and 50 times as high as the number of urethane groups. Most preferably, the number of OH groups is between 2 and 20 times as high as the amount of urethane groups.

In another preferred embodiment, the transesterification is carried out in a continuous process. Optionally, the diurethane of the general formula (3) can be introduced into the transesterification reactor together with a solvent. Preferably the solvent has a standard boiling point ≤ 230 °C. Most preferably, the solvent is R'-OH which may be added to the diurethane or simply remain in the product after the formation of the diruethane in step (I).

During the transesterification, R'-OH is formed. In a preferred embodiment of the invention, this compound is removed via the vapor phase. At the same time, in case solvents other than excess R"-OH are present, they are also removed from the reaction system at this stage. By doing so, the equilibrium reaction is pushed to completion resulting in a high yield of the desired R"-O-CO-NH-R-NH-CO-O-R".

The hydroxy compound R"-OH used for the transesterification can be any hydroxy compound that has standard boiling points ≥ 280 °C. This is required to achieve a good separation of the isocyanate of the general formula (1) from the hydroxy compound R"-OH as a distillate after cleavage of the diurethane of the general formula (5) has been performed. In a preferred embodiment of the invention, the hydroxy compound R"-OH has a standard boiling point between ≥ 280 °C and ≤ 370 °C, preferably between ≥ 285 °C and ≤ 345 °C. Examples of such hydroxy compounds are p-cumylphenol, 1-naphthol, 2-naphthol, 2-phenylphenol, 4-phenylphenol, isomers of nonylphenol, stearyl alcohol or cetyl alcohol. Aromatic hydroxy compounds are advantageous because the corresponding diarylurethanes are more readily cleaved in the subsequent step, while the aliphatic hydroxy compounds are advantageous because the transesterification product is more stable, which facilitates transesterification. Thus, both types of hydroxy compounds are equally preferred.

It is not required to use catalysts in the transesterification reaction, but in order to facilitate the reaction, catalysts can be used. Preferably, the catalysts are compounds of copper, zinc, aluminium, tin, titanium, vanadium, iron, cobalt and/or nickel. Particularly preferred catalysts include copper naphthenate, copper chloride, copper acetate, copper bromide, zinc naphthenate, zinc acetate, zinc oxalate, zinc benzoate, zinc hexylate, zinc dithiocatecholate, zinc dodecylbenzenesulfonate, zinc acetylacetonate, zinc hydroxide, zinc oxide, aluminum alkoxylates, aluminum chloride, tin acetate, tin octoate, dibutyl tin dilaurate, dibutyl tin diphenoxide , titanium oxalate, titanium naphthenate, vandadium acetylacetonate, ferrocene, iron naphthenate, iron octoate, iron acetylacetonate, cobalt naphthenate, bis-triphenylphosphine cobalt nitrate, nickel naphthenate or bis-pyridine nickel nitrate. Particularly preferable catalysts are dibutyl tin dilaurate, iron octoate or tin octoate. It is preferred to use catalysts that are non-volatile or that at least have standard boiling points as high as or higher than standard boiling points of the isocyanate of the general formula (1). That way, they can be separated from the isocyanate in step (III) and/or (IV), preferably together with the hydroxy compound R"-OH.

The cleavage of the diurethane of the general formula (5) in step (III) and the distillative separation of the obtained isocyanate OCN-R-NCO from the generated hydroxy compounds in step (IV) can optionally be carried out simultaneously, if the cleavage reaction is carried out in a suitable reactor as for example a column type reactor. However, it is preferred to separate the steps (III) and (IV) so that cleavage takes place first and then the cleavage products are subjected to the separation step (IV). Both steps can be carried out in a similar manner as described in sections [0371]-[0404] of EP 2 679 575 A1. In a preferred embodiment, the cleavage is a thermolytic cleavage and it is carried out in a thin film evaporator with the hydroxy compound R"-OH and the diisocyanate OCN-R-NCO (6) leaving the evaporator as the gaseous phase and at least part of the liquid effluent which will still contain unreacted carbamic acid ester being recycled to the thin film evaporator and again being exposed to thermolytic cleavage conditions. Optionally, a catalyst can be used in the thermolytic cleavage reaction. Suitable catalysts are compounds of copper, zinc, aluminium, tin, titanium, vanadium, iron, cobalt and/or nickel. Particularly preferred catalysts include copper naphthenate, copper chloride, copper acetate, copper bromide, copper iodide, zinc naphthenate, zinc acetate, zinc oxalate, zinc benzoate, zinc hexylate, zinc dithiocatecholate, zinc dodecylbenzenesulfonate, zinc acetylacetonate, zinc hydroxide, zinc oxide, aluminum alkoxylates, aluminum chloride, tin acetate, tin octoate, dibutyl tin dilaurate, dibutyl tin diphenoxide, titanium oxalate, titanium naphthenate, vandadium acetylacetonate, ferrocene, iron naphthenate, iron octoate, iron acetylacetonate, cobalt naphthenate, bis-triphenylphosphine cobalt nitrate, nickel naphthenate or bis-pyridine nickel nitrate. Particularly preferable catalysts are dibutyl tin dilaurate, iron octoate or tin octoate. If catalysts are used in the transesterification reaction, they may be carried over to the cleavage reactor without the need of separating them from the diurethane. To avoid accumulation of high boiling components in the reaction system, at least a part of the liquid effluent of the cleavage reactor can be purged from the system. It is preferred to carry out the cleavage of the diurethanes in a continuous reaction.

Isocyanate and hydroxy compound formed in step (III) are preferably transferred to the distillation apparatus for the separation step (IV) via the gaseous phase. That way, energy losses during condensation and re-evaporation are avoided. In another preferred embodiment the distillation apparatus is a packed column. Upon distillation, the diisocyanate according to general formula (6) is obtained as the distillate and the bottom product contains mainly the hydroxy compound R"-OH. In a preferred embodiment of the invention, the hydroxy compounds are reused in the transesterification reaction that converts the diurethane of the general formula (3) into the diurethane of the general formula (5), optionally after being subjected to a purification step. Preferably, such a purification step is a washing step or more preferably a distillation step. If a catalyst with a similar boiling point as R"-OH was used in the transesterification, it can be recycled to the transesterification together with the hydroxy compound obtained in the distillation step as a bottom product. A similar boiling point as R"-OH with regard to the present invention is preferably a boiling point that is between the boiling point of the diisocyanate according to general formula (6) and the diurethane compounds present in the thermal cleavage reactor.

### Example 1a (comparative example)

The comparative example 1 is the formation of N,N'-hexanediyl-di(carbamic acid(4-cumylphenyl)ester) according to the method described in example 14 (step 14-1) of EP 2 679 575 A1 on a technical scale.

A first raw material mixture A is prepared that contains 2.9 wt% of HDA, 4.6 wt% of urea and 92.5 wt% of p-cumylphenol. A second raw material mixture B is prepared that contains 7.5 wt% of urea and 92.5 wt% of p-cumylphenol. Mixture A is then introduced into a heated reaction column at a rate of 70 t/h and mixture B is introduced at a rate of 29.3 t/h. Accordingly, the total mass flows of the individual components into the reaction column are 2.0 t/h for HDA, 5.4 t/h for urea and 91.8 t/h for p-cumylphenol. The molar ratio of the compounds is about 25:5:1 (p-cumylphenol:urea:HDA).

The reaction is performed at 2 kPa and 215 °C with removal of ammonia from the reaction system. The desired N,N'-hexanediyl-di(carbamic acid(4-cumylphenyl)ester) is formed in good yield.

### Example 1b (thermal cleavage)

The product of example 1a can be subjected to thermal cleavage which results in the formation of hexamethylenediisocyanate (HDI) and p-cumylphenol. A process for this thermal cleavage is described in example 14 (step 14-3) with reference to example 9 (step 9-3) of EP 2 679 575 A1. The gaseous cleavage products are introduced into a distillation column, where pure HDI is obtained as the distillate whereas p-cumylphenol is contained in the bottom product of the distillation.

### Example 2a (process according to the invention, step (I) according to the present invention)

In a first step, 1,6-hexamethylene-O,O'-diphenylurethane is prepared. The method is again based on the method from example 14 (step 14-1) of EP 2 679 575 A1 to allow better comparison. Of course it is also possible to adapt the methods described in EP 0 320 235 A2. A first raw material mixture C is prepared that contains 6.8 wt% of HDA, 11.6 wt% of urea and 81.6 wt% of phenol. A second raw material mixture D is prepared that contains 11 wt% of urea and 89 wt% of phenol. Mixture C is then introduced into a heated reaction column at a rate of 30 t/h and mixture D is introduced at a rate of 18 t/h. Accordingly the total mass flows of the individual components into the reaction column are, 2.0 t/h for HDA, 5.4 t/h for urea and 40.5 t/h of phenol. The molar ratio of the compounds is about 25:5:1 (phenol:urea:HDA).

The reaction is performed at 2 kPa and 215 °C with removal of ammonia from the reaction system. The desired 1,6-hexamethylene-O,O'-diphenylurethane is formed in good yield.

### Example 2b (transesterification, step (II) according to the present invention)

The product of example 2a can be subjected to a transesterification reaction, adapting methods known from the literature (see for example [0054-0061] of EP 2088 137 B1 or [0347-0370] of EP 2 679 575 A1). For that purpose, the content of 1,6-hexamethylene-O,O'-diphenylurethane in the product mixture from example 2a is determined before it is transferred to a column type transesterification reactor where it is converted with excess amount of p-cumylphenol. Phenol contained in the reaction mixture is removed from the reaction system via the vapor phase in order to drive the equilibrium reaction towards the desired product N,N'-hexanediyl-di(carbamic acid(4-cumylphenyl)ester).

### Example 2c (thermal cleavage & distillation, steps (III) and (IV) according to the present invention)

The product of example 2b can be subjected to thermal cleavage which results in the formation of hexamethylenediisocyanate (HDI) and p-cumylphenol. A process for this thermal cleavage is described in example 14 (step 14-3) with reference to example 9 (step 9-3) of EP 2 679 575 A1. The gaseous cleavage products are introduced into a distillation column, where pure HDI is obtained as the distillate whereas p-cumylphenol is contained in the bottom product of the distillation.

### Example 3a (process according to the invention, step (I) according to the present invention)

In a first step, toluene-2,4-dibutylurethane is prepared. The method is based on the method described in example 18 (step 18-1) of EP 2 679 575 A1. A first raw material mixture E is prepared at 115 °C that contains 7.6 wt% of 2,4-toluene diamine (2,4-TDA), 13.0 wt% of urea and 79.4 wt% of n-butanol. A second raw material mixture F is prepared at 115 °C that contains 13 wt% of urea and 87 wt% of n-butanol. Mixture E is then introduced into a heated reaction column at a rate of 27 t/h and mixture F is introduced at a rate of 11.5 t/h. Accordingly the total mass flows of the individual components into the reaction column are, 2.0 t/h for 2,4-TDA, 5.0 t/h for urea and 31.4 t/h of n-butanol. The molar ratio of the compounds is about 25:5:1 (n-butanol:urea:2,4-TDA). The reaction is performed at 2 kPa and 215 °C with removal of ammonia from the reaction system. The desired toluene-2,4-dibutylurethane is formed in good yield.

### Example 3b (transesterification, step (II) according to the present invention)

The product of example 3a can be subjected to a transesterification reaction, adapting methods known from the literature (see for example [0054-0061] of EP 2088 137 B1 or [0347-0370] of EP 2 679 575 A1). For that purpose, the content of toluene-2,4-dibutylurethane in the product mixture from example 2a is determined before it is transferred to a column type transesterification reactor where it is converted with excess amount of p-cumylphenol. N-butanol is removed from the reaction system via the vapor phase in order to drive the equilibrium reaction towards the desired product toluene-2,4-bis((4-cumylphenyl)urethane).

### Example 3c (thermal cleavage & distillation, steps (III) and (IV) according to the present invention)

The product of example 3b can be subjected to thermal cleavage which results in the formation of 2,4-toluene diisocyanate (TDI) and p-cumylphenol. A suitable process for this thermal cleavage that can be adapted for the different starting material is described in example 9 (step 9-3) of EP 2 679 575 A1. The gaseous cleavage products are introduced into a distillation column, where pure TDI is obtained as the distillate whereas p-cumylphenol is contained in the bottom product of the distillation.

### Discussion of the examples

High stoichiometric excess of urea and aromatic hydroxy compound is required in order to suppress the formation of higher oligomers and/or polymers that would cause fouling inside the reaction system. Therefore, for converting 2.0 t/h of the diamine, a total of 91.8 t/h of the p-cumylphenyl has to be fed to the reactor as shown in example 1a. This is economically unfavorable since the high mass flow is associated with high costs for handling the high material flow. Using the inventive process, the stoichiometric ratios can be kept constant while the mass flows in the urethanization reaction are significantly reduced.

## Claims

1. A process for preparing hexamethylenediisocyanate, pentamethylenediisocyanate or toluenediisocyanate comprising the following steps:
(I) Reacting a primary diamine of the general formula (1), urea and a hydroxy compound of the general formula (2) with removal of ammonia to a diurethane of the general formula (3),
H₂N-R-NH₂ (1)
R'-OH (2) 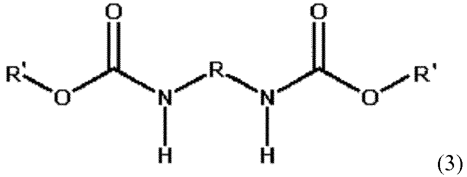
wherein
R represents a bivalent hydrocarbon radical which can be derived from 2,4-toluene diisocyanate, 2,6-toluene diisocyanate, hexamethylene diisocyanate or pentamethylene diisocyanate by removing the two isocyanate groups,
R' represents a hydrocarbon radical which can be derived from an aliphatic or an aromatic hydroxy compound with a standard boiling point ≤ 230 °C by removing the OH group,
(II) Converting the diurethane obtained in step (I) to a diurethane of the general formula (4) by a transesterification reaction with a hydroxy compound of the general formula (5), 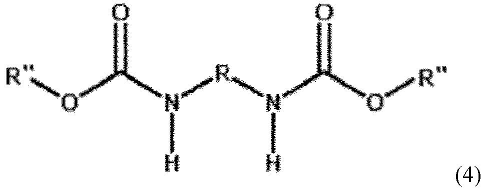
R"-OH (5)
wherein
R is the same as above,
R" represents a hydrocarbon radical which can be derived from an aliphatic or an aromatic hydroxy compound with a standard boiling point ≥ 280 °C by removing the OH group,
(III) Subjecting the diurethane of the general formula (4) to a thermal cleavage reaction to prepare the diisocyanate of the general formula (6)
OCN-R-NCO (6)
wherein
R is the same as above,
and the hydroxy compound of the general formula (5)
(IV) Separating the diisocyanate of the general formula (6) from the hydroxy compound of the general formula (5) by distillation.

2. The process according to claim 1 wherein the molecular mass of R'-OH is at least 40 g/mol lower, preferably at least 100 g/mol lower and most preferably between ≥110 g/mol and < 160 g/mol lower than the molecular mass of R"-OH.

3. The process according to claim 1 or 2 wherein the primary diamine (1) for formation of the diurethanes of the general formula (3) is 1,5-pentanediamine (PDA) or 1,6-hexanediamine (HDA).

4. The process according to any of the claims 1 to 3 wherein the hydroxy compound R'-OH used for the formation of the diurethanes of the general formula (3) that has a standard boiling point between ≥ 80 °C and ≤ 210 °C.

5. The process according to any of the claims 1 to 4 wherein the hydroxy compound R"-OH has a standard boiling point between ≥ 280 °C and ≤ 370 °C

6. The process according to any of the claims 1 to 5, wherein a catalyst is present during the transesterification step.

7. The process according to any of the claims 1 to 6 wherein the cleavage reaction is a thermolytic cleavage that is carried out in a thin film evaporator.

8. The process according to any of the claims 1 to 7 wherein a catalyst is used in the cleavage reaction.
